# EUROPEAN PATENT SPECIFICATION

(11) **EP 3 917 543 B1**
(45) Date of publication and mention of the grant of the patent: **01.07.2026**
(21) Application number: 20749283.6
(22) Date of filing: 31.01.2020
(51) Int. Cl.: A61K 33/06, A61K 31/194, A61K 31/401, A61K 33/00, A61K 33/08, A61K 33/26, A61K 33/30, A61K 45/06, A61P 9/08, A61P 9/12, A61P 21/00

(54) **NITRATE COMPOSITIONS FOR THERAPEUTIC AND NON-THERAPEUTIC USE**
NITRATZUSAMMENSETZUNGEN ZUR THERAPEUTISCHEN UND NICHT-THERAPEUTISCHEN ANWENDUNG
COMPOSITIONS DE NITRATE POUR LEURS UTILISATION THÉRAPEUTIQUE ET NON-THÉRAPEUTIQUE

(30) Priority: 01.02.2019 US 201962800361 P; 25.06.2019 US 201962866540 P
(43) Date of publication of application: 08.12.2021
(73) Proprietor: Thermolife International, LLC, Signal Hill, CA 90755 (US)
(72) Inventor: NIKOLAIDIS, Alexandros, 63080 Nea Kallikratia (GR); KRAMER, Ronald, Phoenix, AZ 85048 (US)
(74) Representative: Barker Brettell LLP
(86) International application number: PCT/US2020/016274
(87) International publication number: WO 2020/160509

(56) References cited:
- WO-A1-2020/214841
- US-A1- 2004 058 011
- US-A1- 2011 123 654
- US-A1- 2018 133 247
- VS KOUZENKOV, AL KRUSHINSKY : "Sodium potassium effect on development of nerological deficiency in experimental model of brain ishemia", MOSCOW UNIVERSITY BULLETIN SER. 16. BIOLOGY, vol. 4, 2014, pages 9 - 14, XP055831436
- UDINTSEV: "Nitrates and physical performance", 9 August 2018 (2018-08-09), pages 1 - 6, XP055831440, Retrieved from the Internet <URL:https://tfzp.ru/zdorovyj-obraz-zhizni/v/nitraty/nitraty-i-fizicheskaya-rabotosposobnost> [retrieved on 20200528]

## Description

### Cross Reference to Related Applications

This application claims the benefit of and priority to U.S. provisional patent application 62/800,361, filed February 1, 2019, and U.S. provisional patent application 62/866,540, filed June 25, 2019.

### Background

Applicants discovered in 2007 that administration of a salt of nitric acid (N0₃⁻, which is also described herein as a nitrate anion) can increase athletic performance, increase blood flow, and improve vasodilation. Since Applicants' discovery, the research community has discovered a multitude of benefits from such inorganic nitrates, for example, the amelioration of exercise fatigue, an increase in the time to exhaustion, a reduction of oxygen consumption during exercise, a reduction of oxygen consumption of subjects with metabolic stress-related diseases, amelioration of disturbed glucose metabolism, a decrease in the body fat percentage (thus usefulness in reducing the likelihood of developing obesity and pre-obesity), amelioration of fatty liver disease, an increase in muscle strength, and the potential to treat stomach ulcers in a non-therapeutic context. Notably, the new flood of nitrate research has shown that contrary to earlier observations of a correlation of ingestion of sodium or potassium of nitrate or nitrite to increase blood pressure, administration of a salt of nitric acid has actually resulted in the reduction of blood pressure.

These discoveries have led to the development of multiple supplement formulations that include nitrate. Applicants discovered the surprising benefit of combining a salt of nitrate with amino acids to improve the amino acid's solubility, pharmacokinetics, and vasodilatory characteristics (U.S. Patent No. 7,777,074). Another group contemplated that use of nitrates with probiotic organisms or polyphenols (U.S. Patent No. 9,180,140). Currently, the most popular forms of inorganic nitrate supplementation on the market are: the nitrate salts of amino acids (disclosed in U.S. Patent No. 7,777,074) and vegetable extracts where the nitrate content is increased through concentration or isolation processes, which also results in the increased bioavailability of the nitrate, when compared to ingestion of just the vegetable.

While the current popularity of searching for beneficial effects of inorganic nitrate has resulted in over 200 clinical trials, it has become clear that certain drawbacks still exits to prevent one from fully taking advantage of the benefits. One such drawback is that nitrate must be administered to the subject hours before its full effects manifest. Multiple groups have found that it takes at least three hours for ingested inorganic nitrate to reach its full beneficial effects on physical activity (U.S. Patent No. 9,180,140; Hoon et al., Int J Sport Nutr Exerc Metab., 2013, 23(5):522-32; and Kapil et al., Hypertension., 2010, 56(2):274-81). Also the dose of nitrate used in most studies to elicit effects on the cardiovascular system has been higher than the current acceptable daily intake of nitrate which currently has an upper limit of 3.7 mg/kg or 277 mg (4.46mmol of inorganic nitrate) for a 75 kg person. This could cause concern with regulatory bodies, raise questions of safety and prohibit the release of properly dosed nitrate products.

Accordingly, there is a need to develop new formulations of inorganic nitrate that contain smaller amount of nitrate and will allow the inorganic nitrate to act faster, ideally increasing the effectiveness per mmole of inorganic nitrate ingested.

The closest prior art is considered to be US 2004/058011 (D1), which discloses topical compositions comprising zinc nitrate and elemental zinc for use as a germicide in cattle. The compositions of the present disclosure differ from those of D1 in that they are formulated for human ingestion and are directed to different therapeutic uses, such as the reduction of blood pressure. Furthermore, the compositions disclosed in D1 are not enabling for human ingestion.

### Summary

The disclosure is directed to compositions for human ingestion comprising an elemental metal and a source of nitrate anion (NO₃⁻). As used herein, "composition for human ingestion" means the composition is suitable for human oral administration; accordingly, nitrate sources that are **poisonous to humans** (e.g., **zinc nitrate)** are excluded from the contemplated compositions. The elemental metal is an alkaline earth metal, an alkali metal, or a transition metal. In some embodiments, the elemental metal is elemental magnesium, elemental calcium, , elemental zinc, or elemental iron. In some aspects, the source of nitrate anion is a salt of nitric acid, for example potassium nitrate, sodium nitrate, or magnesium nitrate. In certain implementations, the composition further comprises an acid. Ingestion of the compositions disclosed herein increase the magnitude as well as the speed in which blood pressure (both diastolic and systolic) is reduced, and this is achieved without ingesting more than the acceptable daily intake level of nitrates.

The disclosure is also directed to methods of reducing blood pressure, treating hypertension, and/or improving athletic performance in a subject. In some aspects of the methods of improving athletic performance in a subject, the aerobic capacity, stamina, muscle strength, endurance, and/or time to exhaustion in a subject are/is increased in a non-therapeutic context. The methods comprise administering to the subject an effective amount of elemental metal and administering to the subject an effective amount of a source of nitrate anion (NO₃⁻). In some implementations, the administered elemental metal is elemental magnesium, elemental calcium, , elemental zinc, or elemental iron. In some implementations, the subject is administered the effective amounts of the elemental metal and the source of nitrate anion (NO₃⁻) via administration to the subject the compositions disclosed herein.

### Brief Description of the Drawings

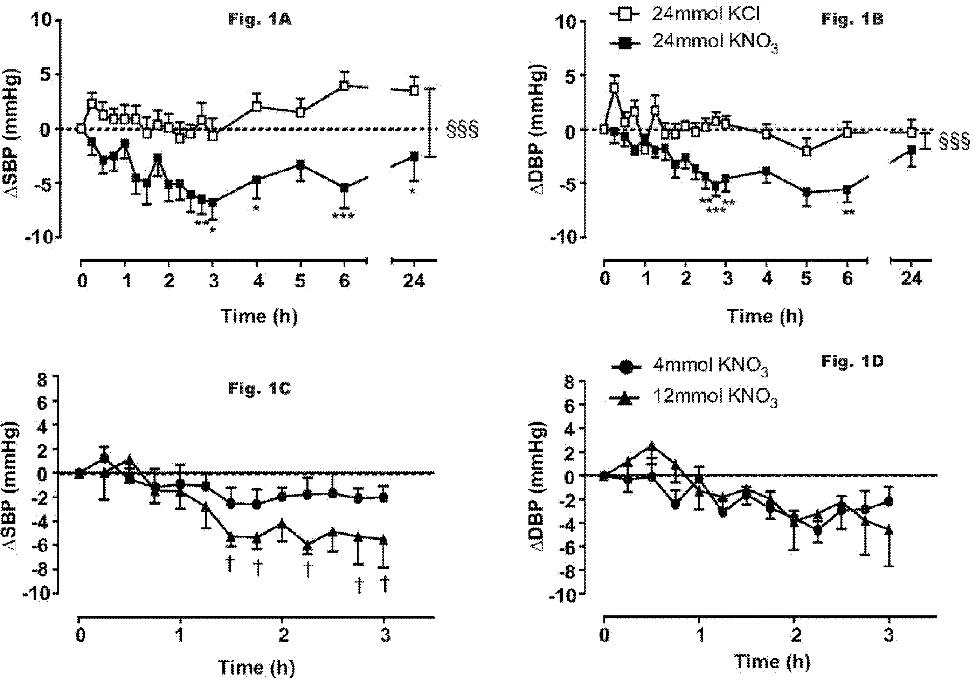

Figs. 1A-D reproduce results from Kapil et al. (Kapil et al., Hypertension., 2010, 56(2):274-81) that demonstrate administration of inorganic nitrate lowers blood pressure. Figs. 1A and 1B depict the effects of 24 mmol KNO₃ on systolic blood pressure and diastolic blood pressure, respectively (n=20, control is 24 mmol KCl). Figs. 1C and 1D depict the effects of lower amount of KNO₃ (4 mmol and 20 mmol) on systolic blood pressure and diastolic blood pressure, respectively (n=6). The data are expressed as mean ± SEM. Statistical significance are shown in comparisons between groups marked by §§§ where P < 0.001 for two-way ANOVA; * where P < 0.05, ** where P < 0.01, and *** where P < 0.001 for Bonferroni post hoc tests; and †where P < 0.05 for one-way ANOVA followed by Dunnett posttest comparison with baseline (t=0).

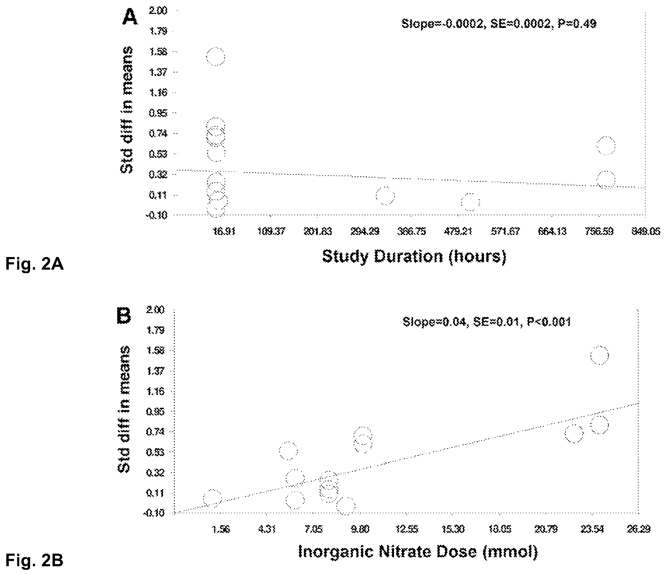

Figs. 2A-B reproduce results from Lara et al., (Lara et al., Eur J Nutr, 2016, 55(2):451-59) reporting that the reports of the effect of inorganic nitrate on endothelial function, while not affected by the duration, is affected by the dosage of the inorganic nitrate administered.

### Detailed Description

Detailed aspects and applications of the disclosure are described below in the following detailed description of the technology. Unless specifically noted, it is intended that the words and phrases in the specification and the claims be given their plain, ordinary, and accustomed meaning to those of ordinary skill in the applicable arts.

In the following description, and for the purposes of explanation, numerous specific details are set forth in order to provide a thorough understanding of the various aspects of the disclosure. It will be understood, however, by those skilled in the relevant art, that implementations of the technology disclosed herein may be practiced without these specific details. It should be noted that there are many different and alternative configurations, devices and technologies to which the disclosed technologies may be applied.

The singular forms "a," "an," and "the" include plural referents unless the context clearly dictates otherwise. Thus, for example, reference to "a step" includes reference to one or more of such steps.

As used herein, the term "acceptable" is a phrase used in its broadest sense and may describe ingredients of a composition that meet Food and Drug Administration (FDA) standards, United States Pharmacopeia (USP) standards, US Department of Agriculture (USDA) standards for food-grade materials, commonly accepted standards of the nutritional supplement industry, industry standards, botanical standards, or standards established by any individual. These standards may delineate acceptable ranges of aspects of ingredients of a composition such as edibility, toxicity, pharmacological effect, or any other aspect of a chemical, composition, or preparation used in implementations of a composition.

As used herein, the term "composition" refers to both a mixture of ingredients or constituents as well as a combination of capsules that contains different ingredients or constituents. Accordingly, in certain embodiments, a composition encompasses separate capsules that are packaged together and are meant to be taken together.

As used herein, the term "elemental metal" refers to the neutral-charged state of a metal element, in other words, a metal in its elemental form and not in a salt form or charged form (exemplary salt forms and charged forms include the oxide, carbonate, chloride, lactate, citrate, aspartate, glycinate, and gluconate of the metal). As such, as used herein, elemental metals and salts of the same metal are different constituents. A description that a composition comprises an elemental metal cannot be satisfied by the presence of a metal salt, and vice versa. For example, a composition that consists of magnesium citrate is not a composition that comprises elemental magnesium in spite of any description that magnesium citrate provides some amount of elemental magnesium. The elemental metals described herein include elemental magnesium, elemental calcium, , elemental zinc, and elemental iron.

The present disclosure relates to compositions with a source of nitrate anion (NO₃⁻) that reduce the time from which the benefits of the nitrate can be observed in a subject. The compositions described herein comprise a source of nitrate anion (NO₃⁻) and an elemental metal. The disclosure also relates to compositions and methods that increase the effectiveness of the nitrate anion (NO₃⁻) in providing a beneficial effect to a subject, for example, causing vasodilation in the subject or a drop in blood pressure in the subject within five minutes or less of administration of the nitrate anion (NO₃⁻) (and thus the disclosed composition). In other aspects, the compositions cause a greater reduction in blood pressure or greater increase in vasodilation in the subject than administration of the same amount of nitrate anion (NO₃⁻) alone. In still other aspects, the compositions cause a greater increase in athletic performance in the subject (for example, as determined through aerobic capacity, stamina, muscle strength, endurance, or time to exhaustion), than administration of the same of nitrate anion (NO₃⁻) alone in a non-therapeutic context.

In some aspects, the source of nitrate anion (NO₃⁻) is a salt of nitric acid. The upper daily intake limit of a salt of nitric acid, which was first established by the World Health Organization, is 3.7 mg inorganic nitrate/kg body weight, which corresponds to an upper limit of 229 mg for the average adult (based on average (global) adult weight of 62 kg) or a total intake of 3.7 mmol inorganic nitrate.

As shown in Figs. 1A-D and Fig. 2B, the majority of studies demonstrating the ability of orally administered inorganic nitrate to reduce both diastolic and systolic blood pressure administered supplementary nitrate at doses higher than the acceptable daily intake level. As reported by Kapil et al., the effective doses of inorganic nitrate for reducing systolic and diastolic blood pressure are well over the acceptable daily intake amount, which could raise questions on the safety of such use of inorganic nitrates. Also concerning is the fact that even when administered with such high amounts of inorganic nitrate, the average reduction in blood pressure during resting state was only 4.1 mm Hg for systolic blood pressure and 2.0 mm Hg for diastolic blood pressure.

Although magnesium supplementation has long been used for purported benefits similar to that of inorganic nitrate, for example, to reduce blood pressure, increase athletic performance, or prevent cramping and increase strength, a meta-analysis of studies on magnesium supplementation (10 mmol (240 mg) of magnesium ingested daily) found that its effect on reducing blood pressure was only an average of 0.6 mm Hg for systolic blood pressure and 0.8 mm Hg for diastolic blood pressure (Jee et al., Am J Hypertens. 2002, 15(8):691-6.). Thus, if the effects on blood pressure of magnesium and inorganic nitrate were additive, one would only expect a reduction of up to 4.3 mm Hg for systolic blood pressure and 2.3 mm Hg for diastolic blood pressure. It was surprisingly discovered that the combination of elemental magnesium (Mg) with a source of nitrate anion (NO₃⁻) resulted in greater and more rapid reduction in blood pressure than the administration of nitrate alone or the typical form of magnesium supplementation alone.

Virtually all of magnesium supplementation is done in the form of magnesium salts such as magnesium citrate, magnesium aspartate, or in the form of magnesium oxide. This is because elemental magnesium is expensive compared to the aforementioned magnesium salts and other salt forms that are found in nature. Elemental magnesium is also eschewed as a form of magnesium supplement due to safety and stability concerns. Small amounts of aggregated elemental magnesium, such as in powdered form or shaved in thin strips, are highly flammable, though it is difficult to ignite in mass or bulk. Elemental magnesium can also readily react with water or moisture in the air to release highly reactive and flammable gases, such as hydrogen. However, as disclosed herein, elemental magnesium has been successfully and safely incorporated into a composition with a salt of nitrate and administered to human subjects.

The administration of the elemental magnesium with a source of nitrate anion (NO₃⁻) resulted in reductions of up to 30 mm Hg reduction in systolic blood pressure and diastolic blood pressure (see Experiments 1-9). Within minutes of ingestion of the described compositions, the subjects reported a feeling of better blood flow throughout the body. In contrast, subjects administered the combination of a salt of inorganic nitrate with the typical salts for magnesium supplementation did not report a similar sensation. The feeling of better blood flow has been referred by athletes has "pump," and while partially psychological, the mere feeling of the composition having some effect can sufficiently enhance the physiological benefits of the composition.

It was also surprisingly discovered that co-administration of a source of nitrate anion (NO₃⁻) with other elemental metals (for example, elemental calcium and elemental zinc) also increased the effectiveness of the nitrate anion (NO₃⁻), particularly in reducing blood pressure. While calcium supplementation is not expected to have any effect on blood pressure, co-administration of elemental calcium and an amount of a source of nitrate anion (NO₃⁻) that would not have caused changed in blood pressure alone actually resulted in reduced diastolic blood pressure, systolic blood pressure, and mean blood pressure within an hour of administration (see Experiment 10). Zinc in high doses has actually been shown to increase blood pressure. However, when elemental zinc co-administered with the same low amount of a source of nitrate anion (NO₃⁻), diastolic blood pressure, systolic blood pressure, and mean blood pressure were also reduced within an hour of administration (see Experiment 10).

As shown in the Examples, physiological effects from the ingestion of the disclosed compositions occurred within 15 minutes and as early as 5 minutes after ingestion of the composition. In particular, systolic blood pressure and diastolic blood pressure were reduced within 15 minutes after ingestion of the composition. For systolic blood pressure, the reduction was sustained for at least one hour after administration. In some implementations, the reduction of systolic blood pressure was sustained for at least two hours and even at least six hours. As the co-administration of nitrate anion (NO₃⁻) with an elemental metal enhances the physiological activity of the nitrate anion (NO₃⁻), the disclosure relates to methods of reducing blood pressure, treating hypertension, and/or improving athletic performance in a subject comprising coadministering to the subject elemental metal and a source of nitrate anion (NO₃⁻). In some aspects, methods of improving athletic performance in a subject increases aerobic capacity, stamina, muscle strength, endurance, and/or time to exhaustion in the subject in a non-therapeutic context.

This time frame coincides with when pre-workout supplementals are typically administered. While it is recommended that most pre-workout supplements should be taken around 30 minutes prior to training, users often take their supplements just before training. Thus, the disclosed composition is more compatible with the habits of athletes and other users of pre-workout supplements. Instead of needing to remember to take their nitrate supplements three hours before training in order to maximize the benefits of nitrate supplementation, they only need to ingest the claimed composition a few minutes, for example five minutes, before training. The minimal delay between ingestion of the claimed composition and physiological effects attributed to inorganic nitrates can easily be satisfied by the warmup period at the start of training. Accordingly, in certain implementations of the methods of improving athletic performance or of increasing stamina, muscle strength, endurance, and/or time to exhaustion in a non-therapeutic context, the subject is co-administered elemental metal and a source of nitrate anion (NO₃⁻) within 30 minutes prior to the session of physical activity or training.

Because of the rapid and potent action of the disclosed compositions, these compositions can be used for all situations where nitrate's effects are beneficial and a rapid effect is desired. These situations include, for example, reducing or countering the increase in blood pressure caused by stimulants such as octodrine, amphetamine, methamphetamine, cocaine, caffeine, and dimethylamylamine (DMAA), and as a treatment for subjects with hypertension or in a hypertensive crisis. The compositions disclosed herein are also suitable for improving athletic performance and/or increasing stamina, muscle strength, endurance, and/or time to exhaustion during a session of physical training in a non-therapeutic context.

### The Composition

The composition disclosed herein comprises elemental metal and a source of nitrate anion (NO₃⁻). The elemental metal is an alkaline earth metal or an alkali metal. In some embodiments, the elemental alkaline earth metal is elemental magnesium, elemental calcium, or. In some aspects, the composition comprises an effective amount of the source of nitrate anion (NO₃⁻) and an effective amount of the elemental metal, wherein the effective amount of the elemental metal supports enhances the effectiveness of the nitrate anion (NO₃⁻) in providing a beneficial effect. Accordingly, in some embodiments, the effective amount of the source of nitrate anion (NO₃⁻) is an amount sufficient to cause vasodilation, to reduce blood pressure, or to increase athletic performance in a subject. In other embodiments, the effective amount of the source of nitrate anion (NO₃⁻) in the composition is less than the amount of nitrate anion (NO₃⁻) needed alone to cause vasodilation, to reduce blood pressure, or to increase athletic performance in a subject, while the effective amount of the elemental metal is an amount sufficient to support the source of nitrate anion (NO₃⁻) in causing vasodilation, reducing blood pressure, or increasing athletic performance in a subject.

In certain embodiments, the composition comprises 1 to 900 mg elemental magnesium and a source of nitrate anion (NO₃⁻), wherein the source of inorganic nitrate provides 5 to 2000 mg of nitrate anion (NO₃⁻). In some aspects, the source of nitrate anion (NO₃⁻) provides 30 mg to 2000 mg of nitrate anion (NO₃⁻), 50 mg to 2000 mg of nitrate anion (NO₃⁻), 5 mg to 1000 mg of nitrate anion (NO₃⁻), 30 mg to 1000 mg of nitrate anion (NO₃⁻), 50 mg to 1000 mg of nitrate anion (NO₃⁻), 5 mg to 600 mg of nitrate anion (NO₃⁻), 30 mg to 600 mg of nitrate anion (NO₃⁻), 50 mg to 600 mg of nitrate anion (NO₃⁻), 5 mg to 500 mg of nitrate anion (NO₃⁻), 30 mg to 500 mg of nitrate anion (NO₃⁻), or 50 mg to 500 mg of nitrate anion (NO₃⁻). In certain embodiments, the source of nitrate anion (NO₃⁻) is a salt of nitric acid, for example, magnesium nitrate or proline nitrate.

In some aspects, the molar ratio of the source of nitrate anion (NO₃⁻) to the elemental metal in the composition is at least 2:1. The doses of the elemental metal and the source of nitrate anion (NO₃⁻) can be adjusted according to the subject's weight, age, and health status. Typically, normotensive subjects require less of elemental metal and nitrate anion (NO₃⁻) than hypertensive subjects, and hypotensive subjects will require even less elemental metal and nitrate anion (NO₃⁻) than normotensive subjects. In some embodiments, the amount of elemental metal in the composition is between 1 mg and 800 mg or between 5 mg and 400 mg. In some embodiments, the amount of the source of nitrate anion (NO₃⁻) is between 30 mg and 2000 mg or between 50 mg and 600 mg. In one embodiment, the composition comprises 100 mg of elemental metal and 250 mg of magnesium nitrate hexahydrate (corresponds to 60.5 mg of nitrate anion (NO₃⁻) and 23.6 mg of Mg).

The element metal in the composition may be in any form, for example, a powder or granules. The forms of the source of nitrate anion (NO₃⁻) that may be included in the composition are, for example, nitrate salts of amino acids or amino acid derivatives (for example, creatine nitrate, arginine nitrate, carnitine nitrate, n-acetyl carnitine nitrate, citrulline nitrate, and proline nitrate), inorganic nitrate salts (for example, magnesium nitrate, sodium nitrate, potassium nitrate, calcium nitrate, and lithium nitrate, or their mixed salts, cocrystalline formulation and hydrates), or natural nitrate sources. For natural nitrate sources, the nitrate has been concentrated and/or isolated from a natural source, and examples of natural nitrate sources include, but are not limited to, beet juice, beet juice powder, concentrated beet juice powder, celery powder, and red spinach extract. In preferred implementations, the nitrate content of natural nitrate sources is standardized so as to provide the sufficient amount of nitrate. In some aspects, the composition comprises multiple sources of nitrate anion (NO₃⁻).

In some embodiments, the elemental metal in the composition is covered or microencapsulated with a suitable material that is poorly soluble in water but soluble in the acidic environment of the stomach, for example, magnesium oxide, cellulose polymers, alginates (such as calcium alginate), or aluminum hydroxide.

In some embodiments, proline nitrate is the source of nitrate anion (NO₃⁻) in the composition. In some embodiments, the magnesium nitrate is the source of nitrate anion (NO₃⁻) in the composition. In such embodiments, the magnesium nitrate may be anhydrous or hydrated. The degree of hydration of the magnesium nitrate is between one and six molecules of water per molecule of magnesium nitrite. In a particular embodiment, magnesium nitrate hexahydrate is the salt of nitric acid in the composition.

In some embodiments, the disclosed composition further comprises an acid. The acid is added to ensure the pH of the stomach upon ingestion of the claimed composition remains acidic. The acid component can be any acid suitable for human consumption, for example, citric acid, succinic acid, malic acid, ascorbic acid, or tartaric acid. In some aspects, the acid is in a solid form, for example, a powder. Thus, the amount of the acid in the composition in some embodiments is between 50 mg and 20,000 mg, between 50 mg and 2000 mg, between 50 mg and 1000 mg, between 100 mg and 20,000 mg, between 100 mg and 2000 mg, between 100 mg and 1000 mg, between 200 mg and 20,000 mg, between 200 mg and 2000 mg, between 200 mg and 1000 mg, between 300 mg and 20,000 mg, between 300 mg and 2000 mg, between 300 mg and 1000 mg, between 500 mg and 20,000 mg, between 500 mg and 2000 mg, or between 500 mg and 1000 mg. In some aspects, the acid component of the composition is a vinegar. In some implementation, the disclosed composition does not comprise an acid, but the composition is administered with an acid. For example, the composition is co-administered with an acidic solution with pH between 2-6, for example, diluted vinegar or a solution of citric acid. In some aspects, the acidic solution is diluted acetic acid, nitric acid, and/or sulfuric acid.

The disclosed composition may be in the form of a capsule, tablet, pill, liquid, liquid suspension, vapor, powder, granulate, pulverulence, or a combination thereof. In a preferred embodiment, the disclosed composition is in a solid form. In some embodiments, the elemental metal and the source of nitrate anion (NO₃⁻), and in some aspects the acid, are combined into a capsule or a tablet. In other embodiments, the acid is in a separate tablet or capsule than the elemental metal and the source of the nitrate ion. The enhanced activity of co-administration the source of nitrate anion (NO₃⁻) and the elemental metal are not reduced if the subject ingests the source of nitrate anion (NO₃⁻) and the elemental metal separately, for example via co-administration of separate capsules of the source of nitrate anion (NO₃⁻) and the elemental metal. Accordingly, in some aspects, the composition described herein comprises a capsule comprises a source of nitrate anion (NO₃⁻) and a capsule comprising an elemental metal. In embodiments of the composition further comprising an acid, the composition further comprises a capsule comprising the acid.

It is notable that the powdered form of the disclosed composition loses its potency when it is administered in water prior to ingestion. Accordingly, administration of the powdered form of the composition should take care to minimize exposure to water. For example, the subject should ingest the powdered composition and then wash it down with water instead of dissolving the powdered composition in water and drinking the mixture. Also, if the composition is added in an alkaline mixture (pH above 7), such as water mixed with baking soda, it can retain its effectiveness for at least 10 minutes before ingestion, which is typically enough time for a person to mix his pre-workout drink and consume it.

In one embodiment, the composition is one capsule comprising 100 mg of elemental metal, 250 mg of magnesium nitrate hexahydrate (providing 60.5 mg of nitrate anion (NO₃⁻) and 23.6 mg of Mg⁺), and 600 mg of anhydrous citric acid. In another embodiment, the composition comprises 1-2 g Amaranthus (providing 10-90% nitrate), 50-1000 mg vitamin C, 50-1000 mg magnesium oxide, 10-1000 mg L-cysteine, 50-1000 mg theanine, 5-100 mg elemental zinc, 0.5-30 mg folate/5-MTHF, and 1-500 mcg potassium molybdate.

In another embodiment, the composition comprises three capsules, wherein two of the capsules each comprise a source of nitrate anion (NO₃⁻) and the elemental metal while the remaining capsule comprises the acid. For example, the capsule comprising the source of nitrate anion (NO₃⁻) and the elemental metal contains proline nitrate and elemental magnesium, while the capsule comprising the acid contains citric acid. In a particular embodiment, the composition comprises two capsules, each comprising 250-750 mg proline nitrate and 5-200 mg elemental magnesium, and one capsule comprising 250-1250 mg citric acid. As another example, the composition comprises a plurality of capsules that, when combined, provide 1-2 g Amaranthus (providing 10-90% nitrate), 50-1000 mg vitamin C, 50-1000 mg magnesium oxide, 10-1000 mg L-cysteine, 50-1000 mg theanine, 5-100 mg elemental zinc, 0.5-30 mg folate/5-MTHF, and 1-500 mcg potassium molybdate. In a preferred implementation, the vitamin C is in a separate capsule as the other constituents.

In some aspects, the composition further comprises a suitable pharmaceutically acceptable coating to prevent moisture from getting in the tablets and/or an additive. Non-limiting examples of the pharmaceutically acceptable coating include waxes, polymers, solid fatty acids. Non-limiting examples of the additive include a carrier, excipient, binder, colorant, flavoring agent, preservative, buffer, diluent, and combinations thereof. In some aspects, the additive is a pharmaceutically acceptable additive or an acceptable food additive.

### Examples

The disclosure is further illustrated by the following examples that should not be construed as limiting.

Blood pressure of all subjects were measured using a standard electronic cuff blood pressure meter. The blood pressure was measured while the subject was at rest. For all of the subjects, with the exception of the subject in Example 2, their blood pressure was measured with the subject seated.

### Experiment 1:

A 35-year-old moderately hypertensive male subject was orally administered a capsule containing 100 mg of elemental magnesium, 250 mg of magnesium nitrate, and 650 mg of citric acid. His blood pressure was taken before ingesting the capsule and 15, 30, 45, 60, and 120 minutes after taking the capsule, and the results are recorded in the table below. Administration of the composition resulted in a rapid decrease in blood pressure of up to 33 mm Hg (24%) in systolic blood pressure and 46 mm Hg (45%) in diastolic blood pressure. While the reduce systolic blood pressure easily lasted for an hour, the reduced diastolic blood pressure lasted for at least two hours.

| Time after Administration (min) | Systolic Blood Pressure | | Diastolic Blood Pressure | |
|---|---|---|---|---|
| | (mm Hg) | % change | (mm Hg) | % change |
| 0 | 136 | | 103 | |
| 15 | 113 | -17% | 72 | -30% |
| 30 | 126 | -7% | 70 | -32% |
| 45 | 103 | -24% | 57 | -45% |
| 60 | 120 | -12% | 61 | -41% |
| 120 | 137 | 1% | 78 | -24% |

### Experiment 2

A 55-year-old normotensive male subject was given a capsule containing 200 mg magnesium nitrate, 50 mg elemental magnesium and 350 mg of citric acid. His blood pressure taken while the subject laid flat on bed before ingesting the capsule and 15, 30, 45, 60, and 120 minutes after taking the capsule. His blood pressure measurements are recorded in the table below. A maximum reduction of 33 mm Hg (25%) was observed in the subject's systolic blood pressure, and the drop of systolic blood pressure was maintained for at least 6 hours.

| Time after Administration (min) | Systolic Blood Pressure | | Diastolic Blood Pressure | |
|---|---|---|---|---|
| | (mm Hg) | % change | (mm Hg) | % change |
| 0 | 130 | | 69 | |
| 15 | 97 | -25% | 64 | -7% |
| 30 | 109 | -16% | 72 | 4% |
| 45 | 105 | -19% | 70 | 1% |
| 60 | 97 | -25% | 59 | -14% |
| 360 | 101 | -22% | 73 | 6% |

### Experiment 3

A highly trained 27-year-old male weighing 270 lbs was orally administered a capsule containing 150 mg of elemental magnesium, 300 mg magnesium nitrate hexahydrate, and 550 mg citric acid. His blood pressure measurements are recorded in the table below. A maximum reduction of 38 mm Hg (35%) was observed in diastolic blood pressure, and reduction of 28 mm Hg (21%) was observed for systolic blood pressure. The reduction was sustained for at least an hour.

| Time after Administration (min) | Systolic Blood Pressure | | Diastolic Blood Pressure | |
|---|---|---|---|---|
| | (mm Hg) | % change | (mm Hg) | % change |
| 0 | 135 | | 89 | |
| 15 | 116 | -14% | 75 | -16% |
| 10 | 124 | -8% | 73 | -18% |
| 30 | 107 | -21% | 58 | -35% |
| 45 | 110 | -19% | 71 | -20% |
| 60 | 133 | -1% | 61 | -31% |

### Experiment 4

A normotensive 33-year-old female weighing 120 lbs was given a capsule containing 25 mg elemental magnesium, 100 mg magnesium nitrate hexahydrate, and 900 mg citric acid. Her blood pressure measurements are recorded in the table below. In spite of the relatively low dose of magnesium, a notable reduction in blood pressure of up to 10 mm Hg (10%) for systolic blood pressure and 9 mm Hg (13%) for diastolic blood pressure were still observed.

| Time after Administration (min) | Systolic Blood Pressure | | Diastolic Blood Pressure | |
|---|---|---|---|---|
| | (mm Hg) | % change | (mm Hg) | % change |
| 0 | 98 | | 67 | |
| 15 | 91 | -7% | 60 | -10% |
| 30 | 88 | -10% | 58 | -13% |
| 45 | 94 | -4% | 73 | 9% |
| 60 | 85 | -13% | 65 | -3% |

### Experiment 5

To examine whether elemental magnesium is critical to reducing the time for a reduction of blood pressure, a capsule containing 500 mg magnesium nitrate hexahydrate and 500 mg citric acid and a separate capsule containing magnesium oxide and magnesium citrate to provide a total of 400 mg of magnesium orally administered to a 32-year-old normotensive female at the same time. The total magnesium dose of both capsules is 435 mg. Her blood pressure measurements are recorded in the table below. Despite the much higher dose of nitrate and magnesium in the administered capsule compared to the other capsules tested, the drop in blood pressure experienced was less and less sustained.

| Time after Administration (min) | Systolic Blood Pressure | | Diastolic Blood Pressure | |
|---|---|---|---|---|
| | (mm Hg) | % change | (mm Hg) | % change |
| 0 | 112 | | 74 | |
| 15 | 101 | -10% | 70 | -5% |
| 30 | 101 | -10% | 65 | -12% |
| 45 | 110 | -2% | 69 | -7% |
| 60 | 96 | -14% | 73 | -1% |

### Experiment 6

To examine whether having any chemical reactions between the inorganic nitrate and the elemental magnesium take place in the stomach is critical for observed effects on blood pressure, the elemental magnesium is mixed with the inorganic nitrate in water prior to administration to a subject. Specifically, 100 mg of elemental magnesium, 300 mg of magnesium nitrate hexahydrate, and 1 g of citric acid were added in water, stirred well for 5 minutes prior to administering the mixture to a male subject. His blood pressure measurements are recorded in the table below.

| Time after Administration (min) | Systolic Blood Pressure | | Diastolic Blood Pressure | |
|---|---|---|---|---|
| | (mm Hg) | % change | (mm Hg) | % change |
| 0 | 122 | | 62 | |
| 5 | 120 | -2% | 78 | 26% |
| 15 | 115 | -6% | 90 | 45% |
| 30 | 115 | -6% | 67 | 8% |
| 45 | 114 | -7% | 76 | 23% |

### Experiment 7

To test the effect on muscle endurance and muscle hyperemia caused by exercise, bicep circumference of a highly trained, right-handed 27-year-old male was measured before and after exercise until exhaustion and both with and without administration of a capsule containing 100 mg elemental magnesium, 400 mg magnesium nitrate hexahydrate, and 500 mg citric acid. His left and right biceps before exercise had a circumference of 46 cm and 45 cm, respectively, before exercise. The subject was direct to perform bicep curls with his right arm using a 50 lb dumbbell until exhaustion. He managed 19 repetitions, and his right bicep circumference increased to 46 cm (increase of 1 cm or 2.2%) due to muscle hyperemia. The subject was orally administered the capsule containing elemental magnesium and nitrate. He was asked to repeat the bicep curl exercise with his left arm 15 minutes after administration of the capsule. The subject managed 24 bicep curls with this left arm after administration of the capsule. His left bicep circumference after the exercise was 47.5 cm (increase of 1.5 cm or 3.3%). Thus, administration of the elemental magnesium-inorganic nitrate supplement resulted in a 50% greater increase in bicep circumference due to exercise induced hyperemia.

### Experiment 8

Maximum weight lifted and the time to exhaustion on a cycling machine of a highly trained 37 year old 240 lbs male were compared between the subject having no supplementation with a composition described herein (baseline) and the subject having ingested a capsule containing 150 mg elemental magnesium, 350 mg magnesium nitrate hexahydrate, and 500 mg citric acid 15 minutes before performing the exercises. A one-week washout period separated the data collection for the baseline line and the effects of ingesting the capsule. For the cycling exercise, the time for the subject to reach exhaustion with the machine set to highest difficult was recorded.

| | Baseline | With Capsule | Amount Changed | % Change |
|---|---|---|---|---|
| Seated barbell, shoulder (lb) | 280 | 310 | 30 | 14.3 |
| Bench chest press (lb) | 270 | 290 | 20 | 7.4 |
| Biceps dumbbell curls (lb) | 110 | 120 | 10 | 9.1 |
| Cycling (min) | 15 | 20 | 5 | 33.3 |

### Experiment 9

To test whether elemental magnesium can affect blood pressure alone, a capsule containing 100 mg of elemental magnesium was given to a 35-year-old normotensive male subject. His blood pressure was taken before ingesting the capsule and 5, 15, 30, 45 and 60 minutes after taking the capsule. The results are recorded in the table below. Elemental magnesium alone only produced a slight reduction in systolic blood pressure (maximum reduction of 14 mm Hg (12%) at 15 mins after administration), and the reduction was short lived. Surprisingly, diastolic blood pressure was increased for most of the period of observation.

| Time after Administration (min) | Systolic Blood Pressure | | Diastolic Blood Pressure | |
|---|---|---|---|---|
| | (mm Hg) | % change | (mm Hg) | % change |
| 0 | 121 | | 62 | |
| 5 | 107 | -12% | 69 | 11% |
| 15 | 106 | -12% | 59 | -5% |
| 30 | 111 | -8% | 74 | 19% |
| 45 | 124 | 2% | 60 | -3% |
| 60 | 113 | -7% | 75 | 21% |

### Experiment 10

To determine whether elemental magnesium could be substituted for other metals in their elemental form, a capsule containing 100 mg elemental calcium, 250 mg of magnesium nitrate hexahydrate (providing 60.5 mg of inorganic nitrate and 23.6 mg of magnesium), and 600 mg anhydrous citric acid was formulated. The capsule administered to a 35-year-old male normotensive subject weighing 220 lbs. His blood pressure measurements are recorded in the table below.

| Time after Administration (min) | Systolic Blood Pressure | | Diastolic Blood Pressure | | Mean Blood Pressure | |
|---|---|---|---|---|---|---|
| | (mm Hg) | % change | (mm Hg) | % change | (mm Hg) | % change |
| 0 | 136 | | 65 | | 100.5 | |
| 10 | 121 | -11% | 67 | 3% | 94 | -6% |
| 15 | 130 | -4% | 60 | -8% | 95 | -5% |
| 30 | 117 | -14% | 67 | 3% | 92 | -8% |
| 45 | 117 | -14% | 58 | -11% | 87.5 | -13% |
| 60 | 127 | -7% | 63 | -3% | 95 | -5% |

As can be seen there was again a rapid and pronounced decrease of systolic blood pressure, an overall decrease in diastolic blood pressure and a sustained decreased in mean blood pressure. It should be noted that calcium itself, unlike magnesium, has no notable effects on blood pressure even at doses of 1.5 g per day (Weinberger et al., *Am J Hypertens.* 1993, 6(9):799-805). Additionally, the doses of nitrate and magnesium provided in this supplement too low, based on previous reports, to be able to have any effect on blood pressure.

On a separate day, a capsule containing 100 mg elemental zinc, 250 mg magnesium nitrate hexahydrate, and 600 mg citric acid was also formulated and administered to the same subject. His blood pressure measurements after administration of the zinc-containing capsule are recorded in the table below.

| Time after Administration (min) | Systolic Blood Pressure | | Diastolic Blood Pressure | | Mean Blood Pressure | |
|---|---|---|---|---|---|---|
| | (mm Hg) | % change | (mm Hg) | % change | (mm Hg) | % change |
| 0 | 139 | | 68 | | 103.5 | |
| 10 | 124 | -11% | 67 | -1% | 95.5 | -8% |
| 45 | 108 | -22% | 62 | -9% | 85 | -18% |
| 60 | 127 | -9% | 63 | -7% | 95 | -8% |

Despite zinc known to increase blood pressure in high doses, administration of the capsule resulted in reduced systolic blood pressure, diastolic blood pressure, and mean blood pressure. Accordingly, co-administration of an elemental metal with an inorganic nitrate greatly increases the effectiveness of the inorganic nitrate in reducing blood pressure.

### Experiment 11

Ten healthy subjects were administered a composition comprising 100 mg elemental magnesium, 1 g proline nitrate, and 1 g citric acid five minutes prior to exercise. Blood flow to their muscles and vasodilation was assessed through Doppler ultrasound measurement and compared to the same measurement when the subjects were not administered the composition (see table below).

| Time After Exercise | Average Blood Flow | | | Vasodilation | | |
|---|---|---|---|---|---|---|
| (min) | (IU) | % change | | (IU) | % change | |

| Baseline | | | | | | |
|---|---|---|---|---|---|---|
| 0 - no administration | 4.9 | - | - | 60.2 | - | - |
| 0 - post-administration | 5.2 | 6% | - | 61.3 | 2% | - |
| 15 repetitions | 5.2 | 6% | 0% | 141.9 | 136% | 131% |
| 45 repetitions | 5.6 | 14% | 8% | >150 | >149% | >145% |
| 15 | 5.4 | 10% | 4% | 75.0 | 25% | 22% |
| 30 | 5.5 | 12% | 6% | 68.9 | 14% | 12% |
| 45 | 5.2 | 6% | 0% | 76.1 | 26% | 24% |
| 60 | 5.2 | 6% | 0% | 92.5 | 54% | 51% |
| 75 | 5.2 | 6% | 0% | 71.6 | 19% | 17% |
| 90 | 5.2 | 6% | 0% | 75.0 | 25% | 22% |
| 105 | 5.1 | 4% | -2% | 82.2 | 37% | 34% |
| 120 | 5.1 | 4% | -2% | 86.0 | 43% | 40% |
| 135 | 5.1 | 4% | -2% | 74.0 | 23% | 21% |
| 150 | 5.1 | 4% | -2% | 71.6 | 19% | 17% |
| 165 | 5.1 | 4% | -2% | 76.6 | 27% | 25% |
| 180 | 5.0 | 2% | -4% | 78.1 | 30% | 27% |

The composition when administered 5 min before exhaustive exercise increased blood flow from 4.9 IU (baseline) to 5.2 IU (post-administration and before exercise) to 5.6 IU (post-exercise), with values continuing to be over 5.0 IU throughout the rest of the monitoring period. A similar trend was observed for vasodilation (brachial artery diameter). Accordingly, the effects of the composition on blood flow and vasodilation are strong and long-lasting.

### Experiment 12

Twelve subjects were given a composition comprising 100 mg elemental magnesium, 1 g proline nitrate, and 1 g citric acid. Administration of the composition to the subjects increased their aerobic capacity, time to exhaustion, and muscle strength in a statistically significant amount in just 5 minutes after administration. The amount of increased in aerobic capacity, time to exhaustion, and muscle strength were much greater than the amount of increase from administration of just inorganic nitrates.

### References Cited

- Hoon et al., Int J Sport Nutr Exerc Metab., 2013, 23(5):522-32.
- Jee et al., Am J Hypertens. 2002, 15(8):691-6.
- Kapil et al., Hypertension., 2010, 56(2):274-81.
- Lara et al., Eur J Nutr, 2016, 55(2):451-59.
- Weinberger et al., Am J Hypertens. 1993, 6(9):799-805.

## Claims

1. A composition for human ingestion comprising:
elemental metal selected from the group consisting of: elemental magnesium, elemental calcium, elemental zinc, and elemental iron; and
a source of nitrate anion (NO3-).

2. The composition of claim 1, further comprising an acid.

3. The composition of claim 1 or 2, wherein the composition comprises:
between 1 mg and 800 mg of the elemental metal; and
the source of nitrate anion (NO3-) provides between 30 mg and 2000 mg of nitrate anion (NO3-).

4. The composition of claim 1 or 2, wherein the composition comprises:
between 5 mg and 400 mg of the elemental metal; and
the source of nitrate anion (NO3-) provides between 50 mg and 600 mg of nitrate anion (NO3-).

5. The composition of any one of claims 1-4, wherein the elemental metal is elemental magnesium.

6. The composition of any one of claims 2-5, wherein the composition comprises between 50 mg to 20,000 mg of the acid.

7. The composition of any one of claims 1-6, wherein the composition is solid.

8. The composition of any one of claims 1-7, wherein the composition is a dietary supplement.

9. The composition of claim 7 or 8, wherein the composition further comprises a pharmaceutically acceptable additive.

10. The composition of any one of claims 1-9, wherein the source of nitrate anion (NO3-) is magnesium nitrate, potassium nitrate, sodium nitrate, or proline nitrate.

11. The composition of any one of claims 1-10 for use in the reduction of blood pressure in a subject or use in the treatment of hypertension in a subject.

12. The non-therapeutic use of the composition of any one of claims 1-10 in a method of improving athletic performance and/or increasing stamina, muscle strength, endurance, and/or time to exhaustion in a subject during a session of physical training, wherein the elemental metal and a source of nitrate anion (NO3-) are to be orally co-administered to the subject within 30 minutes prior to the session of physical training.

## Patentansprüche

1. Zusammensetzung zur menschlichen Einnahme, umfassend:
elementares Metall, ausgewählt aus der Gruppe, bestehend aus: elementarem Magnesium, elementarem Kalzium, elementarem Zink und elementarem Eisen; und
eine Quelle des Nitratanions (NO3-).

2. Zusammensetzung nach Anspruch 1, ferner umfassend eine Säure.

3. Zusammensetzung nach Anspruch 1 oder 2, wobei die Zusammensetzung Folgendes umfasst:
zwischen 1 mg und 800 mg des elementaren Metalls; und die Quelle des Nitratanions (NO3-) stellt zwischen 30 mg und 2000 mg des Nitratanions (NO3-) bereit.

4. Zusammensetzung nach Anspruch 1 oder 2, wobei die Zusammensetzung Folgendes umfasst:
zwischen 5 mg und 400 mg des elementaren Metalls; und
die Quelle des Nitratanions (NO3-) zwischen 50 mg und 600 mg des Nitratanions (NO3-) bereitstellt.

5. Zusammensetzung nach einem der Ansprüche 1 bis 4, wobei das elementare Metall elementares Magnesium ist.

6. Zusammensetzung nach einem der Ansprüche 2 bis 5, wobei die Zusammensetzung zwischen 50 mg und 20.000 mg der Säure umfasst.

7. Zusammensetzung nach einem der Ansprüche 1 bis 6, wobei die Zusammensetzung fest ist.

8. Zusammensetzung nach einem der Ansprüche 1 bis 7, wobei die Zusammensetzung ein Nahrungsergänzungsmittel ist.

9. Zusammensetzung nach Anspruch 7 oder 8, wobei die Zusammensetzung ferner einen pharmazeutisch verträglichen Zusatzstoff umfasst.

10. Zusammensetzung nach einem der Ansprüche 1 bis 9, wobei die Quelle des Nitratanions (NO3-) Magnesiumnitrat, Kaliumnitrat, Natriumnitrat oder Prolinitrat ist.

11. Zusammensetzung nach einem der Ansprüche 1 bis 10 zur Verwendung bei der Senkung des Blutdrucks bei einem Subjekt oder zur Verwendung bei der Behandlung von Bluthochdruck bei einem Subjekt.

12. Nicht-therapeutische Verwendung der Zusammensetzung nach einem der Ansprüche 1 bis 10 in einem Verfahren zur Verbesserung der sportlichen Leistung und/oder zur Erhöhung der Ausdauer, der Muskelkraft, der Kraftausdauer und/oder der Zeit bis zur Erschöpfung bei einem Subjekt während einer körperlichen Trainingseinheit, wobei das elementare Metall und eine Quelle für ein Nitratanion (NO3-) dem Subjekt innerhalb von 30 Minuten vor der körperlichen Trainingseinheit gemeinsam oral verabreicht werden sollen.

## Revendications

1. Composition destinée à la consommation humaine, comprenant :
un métal élémentaire choisi parmi le groupe constitué des éléments suivants : le magnésium élémentaire, le calcium élémentaire, le zinc élémentaire et le fer élémentaire ; et
une source d'anions nitrate (NO3-).

2. Composition selon la revendication 1 comprenant en outre un acide.

3. Composition selon la revendication 1 ou 2, dans laquelle la composition comprend :
entre 1 mg et 800 mg de métal élémentaire ; et
la source d'anions nitrate (NO3-) fournit entre 30 mg et 2 000 mg d'anions nitrate (NO3-).

4. Composition selon la revendication 1 ou 2, dans laquelle la composition comprend :
entre 5 mg et 400 mg du métal élémentaire ; et
la source d'anions nitrate (NO3-) fournit entre 50 mg et 600 mg d'anions nitrate (NO3-).

5. Composition selon l'une quelconque des revendications 1-4, dans laquelle le métal élémentaire est du magnésium élémentaire.

6. Composition selon l'une quelconque des revendications 2-5, dans laquelle la composition comprend entre 50 mg et 20 000 mg d'acide.

7. Composition selon l'une quelconque des revendications 1-6, dans laquelle la composition est solide.

8. Composition selon l'une quelconque des revendications 1-7, dans laquelle la composition est un complément alimentaire.

9. Composition selon la revendication 7 ou 8, dans laquelle la composition comprend en outre un additif pharmaceutiquement acceptable.

10. Composition selon l'une quelconque des revendications 1-9, dans laquelle la source d'anion nitrate (NO3-) est le nitrate de magnésium, le nitrate de potassium, le nitrate de sodium ou le nitrate de proline.

11. Composition selon l'une quelconque des revendications 1-10, destinée à être utilisée pour réduire la pression artérielle chez un sujet ou pour traiter l'hypertension chez un sujet.

12. Utilisation non thérapeutique de la composition selon l'une quelconque des revendications 1-10 dans un procédé visant à améliorer les performances sportives et/ou à augmenter l'endurance, la force musculaire, la résistance et/ou le temps avant l'épuisement chez un sujet au cours d'une séance d'entraînement physique, dans laquelle le métal élémentaire et une source d'anions nitrate (NO3-) doivent être co-administrés par voie orale au sujet dans les 30 minutes précédant la séance d'entraînement physique.
